# EUROPEAN PATENT APPLICATION

(11) **EP 4 424 428 A1**
(43) Date of publication of application: **04.09.2024**
(21) Application number: 22886792.5
(22) Date of filing: 18.10.2022
(51) Int. Cl.: B05B 17/00, B05B 17/06, A61L 2/18, A61L 2/24, A61L 9/14

(54) **MOBILE DECONTAMINATION APPARATUS**

(30) Priority: 25.10.2021 JP 2021173765
(71) Applicant: Airex Co., Ltd., Nagoya-shi Aichi 453-0015 (JP)
(72) Inventor: KAWASAKI, Koji, Nagoya-shi, Aichi 453-0015 (JP); KAKUDA, Daisuke, Nagoya-shi, Aichi 453-0015 (JP); OGATA, Yoshitaka, Nagoya-shi, Aichi 453-0015 (JP); KITAHORA, Kazuhiko, Nagoya-shi, Aichi 453-0015 (JP); FUTAMURA, Haruka, Nagoya-shi, Aichi 453-0015 (JP); GUO, Zhiqiang, Nagoya-shi, Aichi 453-0015 (JP); KITANO, Tsukasa, Nagoya-shi, Aichi 453-0015 (JP)
(74) Representative: Kraus & Lederer PartGmbB
(86) International application number: PCT/JP2022/038725
(87) International publication number: WO 2023/074464

(57) **Abstract**

Provided is a mobile decontamination apparatus capable of uniformly and completely decontaminating a room targeted to be decontaminated, equipment in a room, and the like, and particularly effective when decontaminating an inside of the room targeted to be decontaminated having a large area or decontaminating insides of a plurality of rooms targeted to be decontaminated.

The mobile decontamination apparatus includes a decontamination solution discharging device and an indoor mobile device. The decontamination solution discharging device includes: a decontamination solution tank; one or more mist generating devices configured to generate a decontamination solution mist from a decontamination solution; a driving circuit configured to control a drive of the mist generating device; and one or more mist discharge ports configured to discharge the decontamination agent mist generated by the mist generating device into the inside of the room targeted to be decontaminated. The indoor mobile device includes: a travel path disposed inside the room targeted to be decontaminated; and a traveling device configured to cause the decontamination solution discharging device to travel along the travel path.

## Description

### TECHNICAL FIELD

The present invention relates to a mobile decontamination apparatus for decontaminating an inside of an isolator and the like using a decontamination agent mist. More particularly, the present invention relates to a mobile decontamination apparatus for decontaminating an inside of a room targeted to be decontaminated having a large area or decontaminating insides of a plurality of rooms targeted to be decontaminated.

### BACKGROUND ART

In manufacturing fields of manufacturing medical or pharmaceutical products or foods or in medical fields such as a surgical room, it is important to maintain a sterile state in the room. In particular, in decontamination of the sterile room that is a working room intended to manufacture medical or pharmaceutical products, there is a need to complete advanced decontamination validation which conforms to the Good Manufacturing Practice (GMP).

In recent years, hydrogen peroxide gas has widely been adopted to decontaminate a working chamber where a sterile environment is required (hereinafter referred to as a "room targeted to be decontaminated"). Advantageously, hydrogen peroxide gas has a strong sterilization effect, and is inexpensively available and effectively utilized as an environmentally-friendly decontamination gas that is ultimately decomposed into oxygen and water. However, hydrogen peroxide gas has been conventionally available for use in decontamination of a small space, such as individual rooms targeted to be decontaminated, e.g., a sterile room, an isolator, or a glove box. On the other hand, there has been a problem that, in order to adopt the hydrogen peroxide gas into decontamination inside a room targeted to be decontaminated having a large area or a plurality of rooms targeted to be decontaminated, a large amount of the hydrogen peroxide gas of a prescribed concentration must be stably supplied.

In particular, in pharmaceutical manufacturing fields, very large-sized sterile isolators are used, for example , to which equipment for filling pharmaceutical products into vials or the like, equipment for freeze-drying the pharmaceutical products filled in the vials, and the like are connected. In order to maintain a sterile environment inside such large-sized sterile isolator isolators, correspondingly large-sized decontamination equipment is used. As a decontamination gas generator used for such large-sized decontamination equipment, for example, a sterilizing liquid vaporizing device disclosed in Patent Literature 1 listed below is used.

A large amount of hydrogen peroxide gas which has been generated by such large-sized decontamination gas generator is obtained by vaporizing hydrogen peroxide solution, and is established in a state of mixture gas of hydrogen peroxide gas and water vapor. This hydrogen peroxide gas has a low density, and is supplied to a room targeted to be decontaminated, via large diameter ducts. Therefore, in order to stably supply to the room targeted to be decontaminated the hydrogen peroxide gas that is adjusted to a predetermined concentration by the decontamination gas generator, there is a need to fully heat the duct by a heater. If the heating exerted by the heater is insufficient, the supplied hydrogen peroxide gas condenses in the duct, and the supply concentration and supply quantity relative to the room targeted to be decontaminated becomes insufficient. Moreover, there has been a problem that the hydrogen peroxide solution generated by condensation is heated, and the inside of the duct is corroded.

Thus, there has been a problem that, in such large-sized decontamination gas generator, construction of large diameter ducts, heater equipment to heat the ducts and other ducts made of an anti-corrosion material are required to supply a large amount of hydrogen peroxide gas from the decontamination gas generator to the room targeted to be decontaminated.

Accordingly, the inventors has previously proposed a decontamination system disclosed in Patent Literature 2 listed below as means for solving the above-described problems. In this decontamination system, a mixed gas-liquid obtained by mixing a hydrogen peroxide solution and compressed air is supplied to rooms targeted to be decontaminated respectively through small-diameter mixed gas-liquid supplying pipes. Consequently, it is possible to accurately supply a hydrogen peroxide solution to each room targeted to be decontaminated without requiring a construction of large-scale and large-diameter ducts, heater equipment for heating the ducts, and ducts made of an anti-corrosion material. However, each room targeted to be decontaminated requires its own decontamination gas generator, and there is a possibility that costs, such as equipment, chemical solutions, and energy, may become a problem.

On the other hand, the inventors has proposed a decontamination method uses a hydrogen peroxide mist instead of a conventional hydrogen peroxide gas, in a decontamination device disclosed in Patent Literature 3 listed below. In this decontamination device, hydrogen peroxide mist decontamination (it is also referred to as aerosol decontamination) is performed in an ultrasonic vibration field, thereby accomplishing a decontamination effect with a proper amount of decontamination agent supplied to a room targeted to be decontaminated and reducing time for works such as aeration to improve efficiency of decontamination works.

In this method, the decontamination effect can be accomplished with a significantly smaller input amount of hydrogen peroxide, but there may be a problem that a particle size of the hydrogen peroxide mist may be unevenly distributed in the case of a room having a large area targeted to be decontaminated, since airflow is not agitated. This causes unevenness in a reaching distance of the hydrogen peroxide mist, and thereby particularly hydrogen peroxide mist having a large particle size falls within a short distance. In particular, there is a problem that, in a case of a room targeted to be decontaminated that are wide and have a large planar aspect ratio, hydrogen peroxide mist having a large particle size falls near a discharge port of a decontamination device and wets a floor surface of the room. Moreover, there is a problem that, in a case of there is equipment disposed inside a room targeted to be decontaminated or equipment suspended above in the room, a large amount of hydrogen peroxide mist will adhere to a front surface of these equipment, thereby preventing the decontamination agent from being dispersed.

### CITATION LIST

### PATENT LITERATURE

Patent Document 1: JP-A-2003-339829
Patent Document 2: WO-A1-2015-166554
Patent Document 3: JP-A-2020-156970

### SUMMARY OF INVENTION

### TECHNICAL PROBLEM

Accordingly, the present invention has been made in order to solve the problems described above, and an object of the present invention is to provide a mobile decontamination apparatus capable of uniformly and completely decontaminating a room targeted to be decontaminated, equipment in a room, and the like, and particularly effective when decontaminating an inside of the room targeted to be decontaminated having an large area or decontaminating insides of a plurality of rooms targeted to be decontaminated.

### SOLUTION TO PROBLEM

In order to solve the problems described above, the inventors have reached completion of the present invention by moving a discharge port of a decontamination device in order to decontaminate a large area using a hydrogen peroxide mist.

That is, a mobile decontamination apparatus (30) according to the present invention, according to claim 1, is directed to a decontamination device configured to decontaminate an inside of a room targeted to be decontaminated (11) using a decontamination agent mist,
the mobile decontamination apparatus including a decontamination solution discharging device (50) and an indoor mobile device (60), characterized in that
the decontamination solution discharging device includes:
   a decontamination solution tank (54);
   one or more mist generating devices (51) configured to generate a decontamination agent mist from a decontamination solution;
   a driving circuit (52) configured to control a drive of the mist generating device; and
   one or more mist discharge ports (55) configured to discharge the decontamination agent mist generated by the mist generating device into the inside of the room targeted to be decontaminated, and
   the indoor mobile device includes:
      a travel path (61) disposed inside the room targeted to be decontaminated; and
      a traveling device (62) configured to cause the decontamination solution discharging device to travel along the travel path.

Moreover, the present invention, according to claim 2, is directed to the mobile decontamination apparatus according to claim 1, further including
a decontamination solution supply device (40) including an external storage tank configured to store the decontamination solution in advance, and a solution supply pipe (43) for supplying the decontamination solution between the external storage tank (41) and the decontamination solution discharging device, characterized in that
the decontamination solution supply device includes a weighing device and a solution supply pump, and is configured to weigh and supply a predetermined amount of the decontamination solution to the decontamination solution discharging device through the solution supply pipe.

Moreover, the present invention, according to claim 3, is directed to the mobile decontamination apparatus according to claim 2, further including
an operation control device (70) configured to control an operation of the decontamination solution supply device, the decontamination solution discharging device, and the indoor mobile device, characterized in that
the operation control device controls, in accordance with a program predetermined, the weighing device and the solution supply pump included in the decontamination solution supply device, to weigh a predetermined amount of decontamination solution and to adjust an amount of the solution supplied to the decontamination solution discharging device,
controls the driving circuit included in the decontamination solution discharging device to adjust an amount of the mist discharged, and
controls the traveling device included in the indoor mobile device to adjust a position of the decontamination solution discharging device inside the room targeted to be decontaminated,
thereby controlling the position of the room targeted to be decontaminated and an amount of the mist discharged at the position.

Moreover, the present invention, according to claim 4, is directed to the mobile decontamination apparatus according to claim 3, further including
a solution supply station (45) configured to temporarily store the predetermined amount of decontamination solution disposed on a pipeline of the solution supply pipe for communicating between the external storage tank and the decontamination solution discharging device, characterized in that
the operation control device controls a solution supply from the decontamination solution supply device to the solution supply station and a solution supply from the solution supply station to the decontamination solution discharging device.

Moreover, the present invention, according to claim 5, is directed to the mobile decontamination apparatus according to claim 1, characterized in that
the decontamination solution tank included in the decontamination solution discharging device is an exchangeable cartridge-type tank filled with a predetermined amount of decontamination solution.

Moreover, the present invention, according to claim 6, is directed to the mobile decontamination apparatus according to claim 1, characterized in that
the decontamination solution discharging device or the indoor mobile device includes an exchangeable cartridge-type power supply device.

Moreover, the present invention, according to claim 7, is directed to the mobile decontamination apparatus according to any one of claims 1-6, characterized in that
the mist generating device is an ultrasonic atomizer including a piezoelectric vibrator (51a) and a porous vibration plate (51b) with a plurality of micropores for atomizing the decontamination solution by vibration of the piezoelectric vibrator, the micropores provided so as to pass through the porous vibration plate between front and back surfaces thereof, wherein
the porous vibration plate constitutes the mist discharge port.

The reference signs in the parentheses for each of the above-described means indicate a correspondence relationship with concrete means described in embodiments described below.

### ADVANTAGEOUS EFFECTS OF INVENTION

According to the above-described configuration, a mobile decontamination apparatus includes a decontamination solution discharging device and an indoor mobile device. The decontamination solution discharging device includes a decontamination solution tank, one or more mist generating devices configured to generate a decontamination solution mist from a decontamination solution, a driving circuit configured to control a drive of the mist generating device, and one or more mist discharge ports configured to discharge the decontamination agent mist generated by the mist generating device into the inside of the room targeted to be decontaminated. The indoor mobile device includes a travel path disposed inside the room targeted to be decontaminated, and a traveling device configured to cause the decontamination solution discharging device to travel along the travel path.

Consequently, it is possible to provide a mobile decontamination apparatus capable of uniformly and completely decontaminating a room targeted to be decontaminated, equipment in a room, and the like, and particularly effective when decontaminating an inside of the room targeted to be decontaminated having a large area or decontaminating insides of a plurality of rooms targeted to be decontaminated.

Moreover, according to the above-described configuration, the mobile decontamination apparatus includes a decontamination solution supply device including an external storage tank configured to store the decontamination solution in advance, and a solution supply pipe for supplying the decontamination solution between the external storage tank and the decontamination solution discharging device. The decontamination solution supply device includes a weighing device and a solution supply pump, and is configured to weigh and supply a predetermined amount of the decontamination solution to the decontamination solution discharging device through the solution supply pipe. Consequently, the above-described operational advantageous effects can more specifically and more effectively be demonstrated.

Moreover, according to the above-described configuration, the mobile decontamination apparatus may include an operation control device configured to control an operation of the decontamination solution supply device, the decontamination solution discharging device, and the indoor mobile device. Moreover, the operation control device controls, in accordance with a program predetermined, the weighing device and the solution supply pump included in the decontamination solution supply device, to weigh a predetermined amount of decontamination solution and to adjust an amount of the solution supplied to the decontamination solution discharging device. Moreover, the operation control device controls the driving circuit included in the decontamination solution discharging device to adjust an amount of the mist discharged. Moreover, the operation control device controls the traveling device included in the indoor mobile device to adjust a position of the decontamination solution discharging device inside the room targeted to be decontaminated.

Accordingly, a position of the room targeted to be decontaminated and an amount of the mist discharged at the position can be controlled in accordance with the program predetermined in the operation control device. Consequently, the above-described operational advantageous effects can more specifically and more effectively be demonstrated.

Moreover, according to the above-described configuration, the mobile decontamination apparatus may include a solution supply station. This solution supply station is disposed on a pipeline of the solution supply pipe communicating between the external storage tank and the decontamination solution discharging device, and is configured to temporarily store a predetermined amount of decontamination solution. Moreover, the operation control device controls a solution supply from the decontamination solution supply device to the solution supply station and a solution supply from the solution supply station to the decontamination solution discharging device. Consequently, the above-described operational advantageous effects can more specifically and more effectively be demonstrated.

Moreover, according to the above-described configuration, the decontamination solution tank included in the decontamination solution discharging device may be an exchangeable cartridge-type tank filled with a predetermined amount of decontamination solution. Consequently, it is possible to demonstrate the above-described operational advantageous effect more specifically and more effectively not only in the decontamination inside a room targeted to be decontaminated having a large area or a plurality of rooms targeted to be decontaminated, but also in the decontamination of compact isolators, pass boxes, and the like.

Moreover, according to the above-described configuration, the decontamination solution discharging device or the indoor mobile device may include an exchangeable cartridge-type power supply device. Consequently, it is possible to demonstrate the above-described operational advantageous effect more specifically and more effectively not only in the decontamination inside a room targeted to be decontaminated having a large area or a plurality of rooms targeted to be decontaminated, but also in the decontamination of compact isolators, pass boxes, and the like.

Moreover, according to the above-described configuration, the mist generating device may be an ultrasonic atomizer. This ultrasonic atomizer includes a piezoelectric vibrator, and a porous vibration plate with a plurality of micropores for atomizing the decontamination solution by vibration of the piezoelectric vibrator provided so as to pass through the porous vibration plate between front and back surfaces thereof. In addition, the aforementioned porous vibration plate constitutes the mist discharge port. Consequently, the above-described operational advantageous effects can more specifically and more effectively be demonstrated.

### BRIEF DESCRIPTION OF DRAWINGS

FIG. 1(A) is a front view diagram and FIG. 1(B) is a top view diagram, which respectively are internal cross-sectional diagrams illustrating a state in which an inside of a chamber of an isolator device is decontaminated with a hydrogen peroxide mist in accordance with a conventional method;
FIG. 2(A) is a front view diagram and FIG. 2 (B) is a top view diagram, which respectively are internal cross-sectional diagrams illustrating a state in which a mist discharging device and an ultrasonic vibration board are added to the inside of the chamber illustrated in FIG. 1;
FIG. 3(A) is a front view diagram and FIG. 3(B) is a top view diagram, which respectively are internal cross-sectional diagrams illustrating a state in which articles are disposed inside the chamber illustrated FIG. 1;
FIG. 4 (A) is a front view diagram and FIG. 4 (B) is a top view diagram, which respectively are internal cross-sectional diagrams illustrating a state in which articles are disposed inside the chamber illustrated FIG. 2;
FIG. 5 (A) is a front view diagram and FIG. 5 (B) is a top view diagram, which respectively are internal cross-sectional diagrams illustrating a state in which the inside of the chamber of the isolator device is decontaminated with a hydrogen peroxide mist using a mobile decontamination apparatus according to a first embodiment of the present invention;
FIG. 6 (C) is a top view diagram and FIG. 6(D) front view diagram, which respectively are drawings illustrating a decontamination solution discharging device used for the mobile decontamination apparatus illustrated in FIG. 5;
FIG. 7 (E) is a front view diagram observed from the inside of the chamber and FIG. 7 (F) is a side cross-sectional diagram, which respectively are drawings illustrating a mist generating device of the decontamination solution discharging device illustrated in FIG. 6;
FIG. 8 is a top view diagram of an internal cross section illustrating a state in which a room targeted to be decontaminated in a second embodiment is decontaminated by a conventional method.
FIG. 9 is a top view diagram of an internal cross section illustrating a state where the room targeted to be decontaminated in the second embodiment is decontaminated by the mobile decontamination apparatus according to the present invention;
FIG. 10 is a top view diagram of an internal cross section illustrating a state in which a room targeted to be decontaminated in a third embodiment is decontaminated by a conventional method; and
FIG. 11 is a top view diagram of an internal cross section illustrating a state where the room targeted to be decontaminated in the third embodiment is decontaminated by the mobile decontamination apparatus according to the present invention.

### DESCRIPTION OF EMBODIMENTS

First, prior to describing a mobile decontamination apparatus according to the present invention, a conventional decontamination method using a hydrogen peroxide mist will now be described. In the conventional decontamination method and the mobile decontamination apparatus according to the present invention both illustrated below, a hydrogen peroxide solution is used as a decontamination agent. Although a concentration of the hydrogen peroxide solution to be used is not limited in particular, in general, it is preferable to use the hydrogen peroxide solution of 30% to 35% by weight in consideration of handling of a hazardous material or the like. Moreover, the decontamination agent used in the conventional decontamination method and the mobile decontamination apparatus according to the present invention is not limited to the hydrogen peroxide solution, and may be any liquid decontamination agent, such as an aqueous solution of peracetic acid.

The conventional decontamination method and the mobile decontamination apparatus according to the present invention are configured to, in order to decontaminate an inside of rooms targeted to be decontaminated, such as clean rooms, isolators, or an inside of pass boxes connected to these rooms targeted to be decontaminated, or devices targeted to be decontaminated or articles targeted to be decontaminated disposed inside these rooms targeted to be decontaminated, convert a decontamination agent into a fine mist and supply the converted mist to the inside of these rooms targeted to be decontaminated.

In the present invention, the term "mist" is broadly interpreted as a state of a liquid droplet of a decontamination agent refined and floating in the air, a state of a gas and a liquid agent of a decontamination agent in mixture, a state of the decontamination agent to repeat the change in phase between condensation and evaporation of a gas and a droplet, and the like. In terms of particle size as well, the mist is also broadly interpreted to include mists, fogs, and liquid droplets, which can be subclassified.

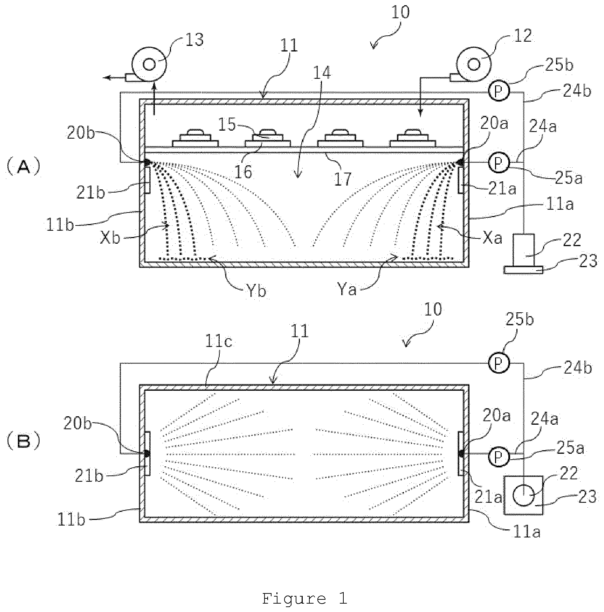

FIG. 1(A) is a front view diagram and FIG. 1(B) is a top view diagram, which respectively are internal cross-sectional diagrams illustrating a state in which an inside of a chamber of an isolator device is decontaminated with a hydrogen peroxide mist in accordance with a conventional method. In FIG. 1, air flows in a unidirectional airflow from above toward below inside the chamber of the isolator device.

In FIG. 1, an isolator device 10 includes a chamber 11, an air supply blower 12, and an air exhaust blower 13 (electrical and mechanical rooms are not illustrated). The chamber 11 is formed with a box configured with a stainless steel metal plate and is airtightly shielded from an external environment in which an operator works except for restricted air intake and exhaust ports. It is assumed that the chamber 11 illustrated in FIG. 1 has a long work area in a right-left direction on the drawing (the length direction is shortened to simplify the description).

Moreover, a ceiling portion inside the chamber 11 is provided with a plurality of air supply fans 15 and filter units 16 for filtering air supplied from the air supply blower 12 to be supplied to a work area 14, and a screen mesh straightening plate 17, disposed at a ceiling portion of the work area 14, for straightening clean air filtered by the air supply filter unit 16 to be supplied to the work area 14.

Inside side face walls 11a, 11b of the chamber 11 (inside the work area 14), mist discharging devices 20a, 20b and ultrasonic vibration boards 21a, 21b are respectively provided at upper portions of internal wall surfaces. Moreover, outside the isolator device 10, an external storage tank 22 and a weighing device 23, and solution supply pipes 24a, 24b and solution supply pumps 25a, 25b are provided for supplying a hydrogen peroxide solution to the mist discharging devices 20a, 20b.

In FIG. 1, the hydrogen peroxide solution mist is discharged from the mist discharging devices 20a, 20b, and is diffused to inside the chamber 11 by ultrasonic waves emitted from the ultrasonic vibration boards 21a, 21b. It is to be noted that the mist discharging devices 20a, 20b and the ultrasonic vibration boards 21a, 21b illustrated in FIG. 1 are both fixed types and are not mobile type, unlike the present invention.

As illustrated in FIG. 1(A), the hydrogen peroxide mist discharged from the mist discharging devices 20a, 20b is falling while diffusing in a parabola. In addition, the hydrogen peroxide mist has particle size distribution, and the hydrogen peroxide mist having large particle sizes (portions Xa, Xb in the drawing) cannot sufficiently reach the position far away from each mist discharging device 20a and 20b, wetting a floor surface in the vicinity thereof (portions Ya, Yb in the drawing). On the other hand, the hydrogen peroxide mist having small particle sizes similarly does not sufficiently reach a center portion of the chamber 11. Such a phenomenon is particularly likely to occur in a case of a chamber that is wide and has a large planar aspect ratio.

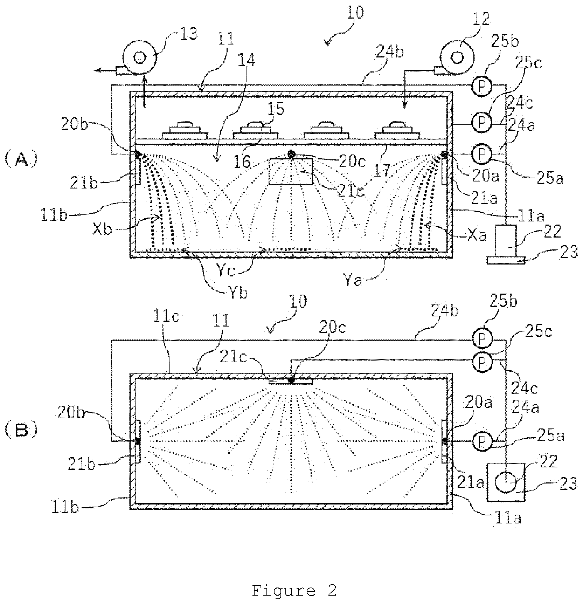

Next, a case will be described in which a mist discharging device is added to the conventional decontamination method using the hydrogen peroxide mist. FIG. 2(A) is a front view diagram and FIG. 2 (B) is a top view diagram, which respectively are internal cross-sectional diagrams illustrating a state in which a mist discharging device and an ultrasonic vibration board are added to the inside of the chamber illustrated in FIG. 1. In FIG. 2, a content of the isolator device 10 and the like is the same as those in FIG. 1, and the same reference signs are used therefor. In FIG. 2, a mist discharging device 20c and an ultrasonic vibration board 21c are added to an upper portion of internal wall surface inside a back surface wall 11c of the chamber 11 (inside the work area 14) . Further, the mist discharging device 20c is provided with a solution supply pipe 24c and a solution supply pump 25c from the external storage tank 22.

In the chamber 11 illustrated in FIG. 2 to which the mist discharging device 20c and the ultrasonic vibration board 21c are added, the hydrogen peroxide mist from the mist discharging device 20c can reach a center portion of the chamber 11. However, due to the particle size distribution of hydrogen peroxide mist, the hydrogen peroxide mist having large particle sizes (portions Xa, Xb, Xc in the drawing, but Xc is not visible) cannot sufficiently reach the position far away from each mist discharging device 20a, 20b, 20c, wetting a floor surface in the vicinity thereof (portions Ya, Yb, Yc in the drawing).

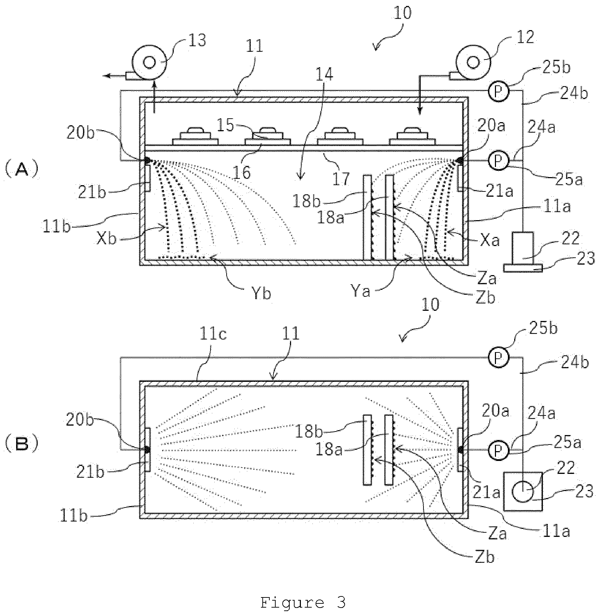

Next, in the conventional decontamination method using the hydrogen peroxide mist, a case will be described in which a device targeted to be decontaminated or an article targeted to be decontaminated is disposed inside the chamber. FIG. 3 (A) is a front view diagram and FIG. 3(B) is a top view diagram, which respectively are internal cross-sectional diagrams illustrating a state in which articles are disposed inside the chamber illustrated FIG. 1. In FIG. 3, a content of the isolator device 10 and the like is the same as those in FIG. 1, and the same reference signs are used therefor. In FIG. 3, articles 18a, 18b are disposed near the sidewall surface 11a inside the chamber 11.

As illustrated in FIG. 3(A), the hydrogen peroxide mist having large particle sizes (portions Xa, Xb in the drawing) discharged from mist discharging devices 20a, 20b cannot sufficiently reach the position far away from each mist discharging device 20a, 20b, wetting a floor surface in the vicinity thereof (portions Ya, Yb in the drawing). Furthermore, the hydrogen peroxide mist having small particle sizes discharged from the mist discharging device 20a also collides with front surfaces of the articles 18a, 18b thereby adhering and condensing thereon, wetting these front surfaces (the portions Za, Zb in the figure) and not reaching the center portion of the chamber 11.

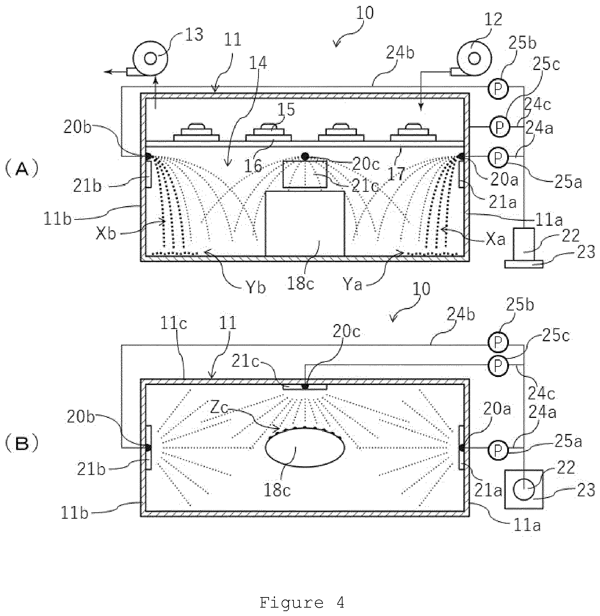

Similarly, FIG. 4(A) is a front view diagram and FIG. 4(B) is a top view diagram, which respectively are internal cross-sectional diagrams illustrating a state in which articles are disposed inside the chamber illustrated FIG. 2. In FIG. 4, a content of the isolator device 10 and the like is the same as those in FIG. 1, and the same reference signs are used therefor. In FIG. 4, an article 18c is disposed at a center portion inside the chamber 11.

As illustrated in FIG. 4 (B), the hydrogen peroxide mist having small particle sizes discharged from the mist discharging device 20c also collides with a front surface of the article 18c, thereby adhering and condensing thereon, wetting the front surface (portion Zc in the figure), and not reaching a surface wall 11d of the chamber 11.

In order to solve the above-described problems, the inventors have developed a mobile decontamination apparatus according to the present invention. Hereinafter, the mobile decontamination apparatus according to the present invention will be described on the basis of each embodiment. It is to be noted that the present invention is not limited to the embodiments shown below.

### <First Embodiment>

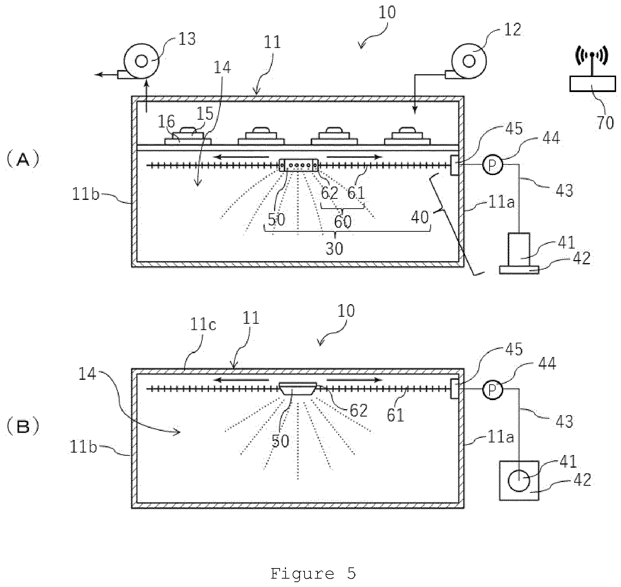

FIG. 5 (A) is a front view diagram and FIG. 5 (B) is a top view diagram, which respectively are internal cross-sectional diagrams illustrating a state in which the inside of the chamber of the isolator device is decontaminated with a hydrogen peroxide mist using a mobile decontamination apparatus according to the first embodiment of the present invention.

In FIG. 5, a content of the isolator device 10 and the like is the same as those in FIG. 1, and the same reference signs are used therefor. In FIG. 5, the mist discharging device and the ultrasonic vibration board provided in the conventional decontamination method illustrated in FIG. 1 are not provided. Instead, a mobile decontamination apparatus 30 is provided at an upper portion of the internal wall surface inside the back surface wall 11c (inside the work area 14) of the chamber 11 and outside the isolator device 10. The mobile decontamination apparatus 30 includes a decontamination solution supply device 40, a decontamination solution discharging device 50, an indoor mobile device 60, and an operation control device 70.

The decontamination solution supply device 40 includes an external storage tank 41, a weighing device 42 and a solution supply pipe 43, a solution supply pump 44, and a solution supply station 45. The external storage tank 41, the weighing device 42, and the solution supply pipe 43 and the solution supply pump 44 are provided outside the isolator device 10. In contrast, the solution supply station 45 is provided at the upper portion of the internal wall surface inside the side face wall 11a (inside the work area 14) of the chamber 11. The external storage tank 41 and the solution supply station 45 are communicated with each other through the solution supply pipe 43. Consequently, a predetermined amount of the hydrogen peroxide solution weighed by the weighing device 42 is supplied to the solution supply station 45 by the solution supply pump 44.

The decontamination solution discharging device 50 discharges the hydrogen peroxide mist while moving inside the work area 14 of the chamber 11. Details of the decontamination solution discharging device 50 will be described later.

The indoor mobile device 60 includes a travel path 61 and a traveling device 62. The travel path 61 is horizontally provided at the upper portion of the internal wall surface inside the back surface wall 11c (inside the work area 14) of the chamber 11, extending from the side face wall 11a to the side face wall 11b. One end (side face wall 11a side) of the travel path 61 is connected to the solution supply station 45. The traveling device 62 is integrally bonded to a back surface of the decontamination solution discharging device 50, and causes the decontamination solution discharging device 50 to travel between the side face wall 11a to the side face wall 11b along the travel path 61.

In the first embodiment, a structure and traveling mechanism of the travel path 61 and the traveling device 62 are not particularly limited. For example, the decontamination solution discharging device 50 may be driven by a mechanical drive such as a servomotor, an ultrasonic motor, or a ball screw, a linear motor drive by a magnetic levitation type, an air drive by a fan, or in some cases by tilting a moving bar. In the first embodiment, a Linear Motion Guide (LM guide) is adopted, an LM rail is used as the travel path 61, and an LM block is used as the traveling device 62.

The operation control device 70 controls each operation of the weighing device 42 and the solution supply pump 44 in the decontamination solution supply device 40, a driving circuit (described latter) of the decontamination solution discharging device 50, the traveling device 62 in the indoor mobile device 60, and the like, and cooperation operations thereof. The control performed by the operation control device 70 will be described latter.

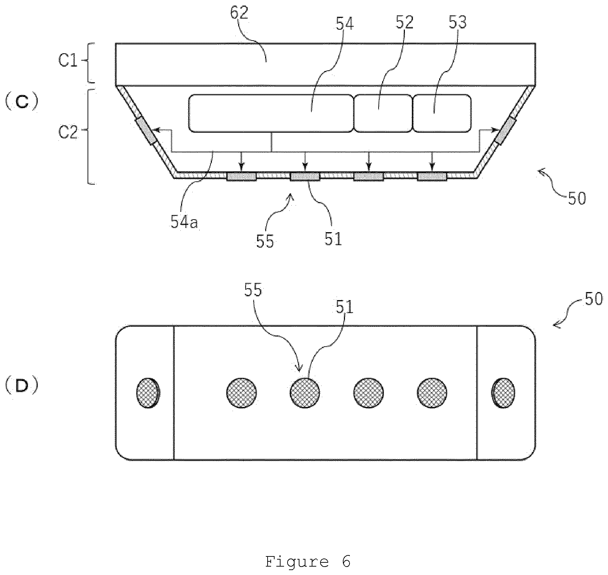

Herein, details of the decontamination solution discharging device 50 will be described. FIG. 6(C) is a top view diagram and FIG. 6(D) front view diagram, which respectively are drawings illustrating a decontamination solution discharging device used for the mobile decontamination apparatus. In FIG. 6(C), the traveling device 62 of the indoor mobile device 60 is integrally bonded to a back surface of the decontamination solution discharging device 50. In FIG. 6(C), a portion C1 is a top view diagram illustrating an outer surface of the traveling device 62, and a portion C2 is a top view diagram illustrating an internal cross section of the decontamination solution discharging device 50. As described above, the traveling device 62 is the LM block of the LM guide and is therefore general, and the detail description thereof is omitted.

In the internal cross-sectional diagram (portion C2) illustrated in FIG. 6(C), the decontamination solution discharging device 50 includes a mist generating device 51, a driving circuit 52, a battery 53, a hydrogen peroxide solution tank 54, and a mist discharge port 55. The mist generating device 51 is operated by the driving circuit 52, converts the hydrogen peroxide solution sent from the hydrogen peroxide solution tank 54 through the narrow tube 54a into the hydrogen peroxide mist, and discharges the hydrogen peroxide mist from the mist discharge port 55 into the inside of the work area 14 in the chamber 11. Temporary storage of the hydrogen peroxide solution in the hydrogen peroxide solution tank 54 is performed when the decontamination solution discharging device 50 travels along the travel path 61 and is connected to the solution supply station 45.

The battery 53 for driving the driving circuit 52 may be an exchangeable cartridge type or rechargeable type. In the case of rechargeable-type battery, any charging method, such as wired or wireless power supply, may be used. Charging to this battery 53 is performed when the decontamination solution discharging device 50 is connected to the solution supply station 45 by the driving of the traveling device 62.

Alternatively, instead of solution supply to the hydrogen peroxide solution tank 54 by the decontamination solution supply device 40, an exchangeable cartridge-type tank filled with the hydrogen peroxide solution may be used as the hydrogen peroxide solution tank 54 provided in the decontamination solution discharging device 50. Thus, by making the battery 53 and/or the hydrogen peroxide solution tank 54 exchangeable cartridge type, there is no need a charging device and/or a decontamination solution supply device, it can be used for decontamination of compact isolators, pass boxes, and the like.

The number of the mist generating devices 51 and the mist discharge ports 55 provided in the decontamination solution discharging device 50 is not particularly limited. In the first embodiment, the decontamination solution discharging device 50 is provided with six mist generating devices 51 and six mist discharge ports 55. As illustrated in FIG. 6, four mist discharge ports 55 are provided on the front of the decontamination solution discharging device 50, and one mist discharge port 55 is provided on each diagonal side surface on both sides.

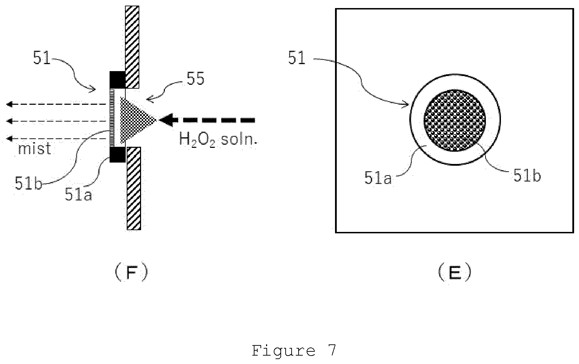

Herein, the mist generating device 51 used in the first embodiment will be described. FIG. 7 (E) is a front view diagram observed from the inside of the chamber and FIG. 7 (F) is a side cross-sectional diagram, which respectively are drawings illustrating a mist generating device of the decontamination solution discharging device. In FIG. 7, the mist generating device 51 is an ultrasonic atomizer including a piezoelectric vibrator 51a and a porous vibration plate 51b, and is fitted into the mist discharge port 55.

The porous vibration plate 51b has a substantially disk-shaped main body with a plurality of micropores for atomizing the hydrogen peroxide solution provided so as to pass through the porous vibration plate between the front and back surfaces thereof. The piezoelectric vibrator 51a is formed in a substantially circular plate shape and is provided at an outer periphery of the porous vibration plate 51b, realizing membrane vibration of the porous vibration plate 51b. The vibration of the piezoelectric vibrator 51a is controlled by the driving circuit 52.

The porous vibration plate 51b is attached with its front surface directed to the inside of the chamber 11, which is a target for decontamination, and its back surface directed to the inside of the decontamination solution discharging device 50. In this state, hydrogen peroxide sent from the hydrogen peroxide solution tank 54 through the narrow tube 54a is discharged from the back surface of the porous vibration plate 51b. FIG. 7 (F) illustrates merely an overview of the discharge and omits piping and the like. Thus, the hydrogen peroxide solution is discharged to the back surface side of the porous vibration plate 51b, and is converted into fine hydrogen peroxide mist through the plurality of micropores of the porous vibration plate 51b which performs ultrasonic vibration, and is discharged to the inside of the chamber 11 that is targeted for decontamination, thereby exerting a decontamination effect.

Herein, the pore diameter and the number of pores of the micropores in the porous vibration plate 51b are not particularly limited, and the diameter and number of pores should be sufficient to ensure the ultrasonic atomization effect and a sufficient supply of hydrogen peroxide mist. It is normally preferable to have a pore diameter of approximately 4 to 11 um. In FIG. 7 (F), although it is disposed so that the hydrogen peroxide mist may be discharged from the front surface of the porous vibration plate 51b in a horizontal direction (leftward in the drawing), it is not limited thereto, and the hydrogen peroxide mist may be discharged downward or upward depending on a disposed position thereof.

The first embodiment adopts an ultrasonic atomizer including the piezoelectric vibrator 51a and the porous vibration plate 51b as the mist generating device 51. However, the mist generation mechanism of the mist generating device is not particularly limited. For example, the mist generation mechanism may be a single-fluid spray nozzle, a piezo high-pressure jet device, an immersion type ultrasonic atomizer, a disk-type atomizer, a disk-mesh type atomizer, or the like for directly forming the solution into mist. Alternatively, it may be an ejector, two-fluid spray nozzle, or the like for form forming the solution into mist by using a high-pressure air or the like.

Next, control of the decontamination solution supply device 40, the decontamination solution discharging device 50, and the indoor mobile device 60 performed by the operation control device 70 will be described with reference to FIG. 5. The control of each device performed by the operation control device 70 may be wireless control or may be wired control. The first embodiment adopts the wireless control.

First, the decontamination solution supply device 40 is operated in accordance with a program predetermined in the operation control device 70. Specifically, the weighing device 42 and the solution supply pump 44 provided in the decontamination solution supply device 40 are operated to weigh a predetermined amount of the hydrogen peroxide solution to be supplied to the solution supply station 45 through the solution supply pipe 43. Herein, the predetermined amount of hydrogen peroxide solution is set in advance in accordance with a shape and capacity of the chamber 11, which is targeted for decontamination, and a capability of the decontamination solution discharging device 50.

Next, the decontamination solution discharging device 50 and the indoor mobile device 60 are operated in accordance with the program predetermined in the operation control device 70. First, the traveling device 62 of the indoor mobile device 60 integrally bonded to the back surface of the decontamination solution discharging device 50 travels along the travel path 61, and the decontamination solution discharging device 50 is contacted with the solution supply station 45 connected to one end (side face wall 11a side) thereof. Herein, the hydrogen peroxide solution tank 54 in the decontamination solution discharging device 50 is filled with the hydrogen peroxide solution from the solution supply station 45. In the first embodiment, wireless power delivery is performed to the battery 53 in the traveling device 62 during this time.

Next, the decontamination solution discharging device 50 travels along the travel path 61 by the operation of the traveling device 62, so as to travel inside the chamber 11 from the side face wall 11a to the side face wall 11b. The travel of the decontamination solution discharging device 50 may be one-way travel or reciprocating travel. Alternatively, it may travel while traversing multiple times. Furthermore, the decontamination solution discharging device 50 may travel at a constant speed or may travel in a manner allowing for acceleration and deceleration. During this time, the decontamination solution discharging device 50 discharges the hydrogen peroxide mist to the inside of the chamber 11, which is a room targeted to be decontaminated, in accordance with the program predetermined in the operation control device 70.

A travelling speed of the decontamination solution discharging device 50 and a discharge amount of the hydrogen peroxide mist are operated in accordance with the program predetermined in the operation control device 70. Moreover, it may be configured to adjust the travelling speed at a predetermined position and the discharge amount of hydrogen peroxide mist in accordance with the shape and capacity of the chamber 11, which is targeted to be decontaminated. The travelling speed of the decontamination solution discharging device 50 is not particularly limited, and may be, for example, several am/sec to tens of cm/sec. On the other hand, the discharge amount of hydrogen peroxide mist is adjusted in accordance with the capacity and position of the chamber 11, the travelling speed of the decontamination solution discharging device 50, and the like, the amount of hydrogen peroxide solution may be, for example, approximately 0.1 to 10 g/min.

In accordance with such a configuration, in the mobile decontamination apparatus related to this first embodiment, even in the case of a chamber that is wide and has a large planar aspect ratio, there is no possibility that hydrogen peroxide mist having large particle sizes fails to sufficiently reach the position far away from the mist generating device. Accordingly, there is no possibility of wetting the floor surface near the mist generating device. On the other hand, there is no possibility that the hydrogen peroxide mist having small particle sizes similarly fails to sufficiently reach the center portion of the chamber. Moreover, even in the state in which an article is disposed inside the chamber, there is no possibility that the hydrogen peroxide mist collides with a surface of the article, thereby adhering and condensing thereon, wetting these front surfaces.

Moreover, since the amount of hydrogen peroxide solution to be supplied to the decontamination solution discharging device can be accurately controlled to the necessary minimum, without the need to install an additional decontamination solution discharging device, efficient decontamination can be performed corresponding to the size of the chamber. Furthermore, since a precise amount of hydrogen peroxide mist can be supplied in this way, a sufficient decontamination effect can be obtained even with the minimum amount of decontamination solution required, achieving efficient use of the decontamination solution.

Accordingly, in the first embodiment, it is possible to provide a mobile decontamination apparatus capable of uniformly and completely decontaminating a room targeted to be decontaminated, equipment in a room, and the like, and particularly effective when decontaminating an inside of the room targeted to be decontaminated having a large area or decontaminating insides of a plurality of rooms targeted to be decontaminated.

### <Second Embodiment>

The second embodiment describes a case in which mobile decontamination apparatus is used to efficiently decontaminate a room targeted to be decontaminated having a complex chamber shape where a plurality of isolator devices are connected.

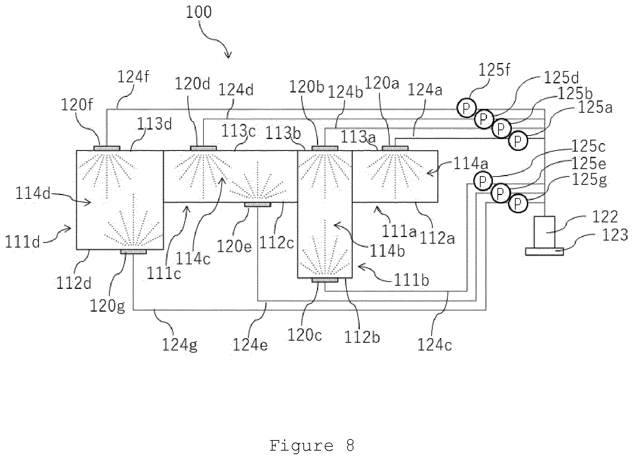

First, a case will be described in which a room targeted to be decontaminated having a complex chamber shape is decontaminated by a conventional method. FIG. 8 is a top view diagram of an internal cross section illustrating a state in which a room targeted to be decontaminated in the second embodiment is decontaminated by a conventional method. In FIG. 8, a room 100 targeted to be decontaminated is formed by complexly combining four chambers 111a to 111d having different shapes and capacities. Boundaries of each chamber are opened to be communicated with each other. Components such as an air supply blower and an air exhaust blower in each chamber are omitted.

Inside front surface walls 112b to 112d and back surface walls 113a to 113d respectively formed in the chambers 111a to 111d (inside work areas 114a to 114d), seven units of fixed decontamination devices 120a to 120g, each of which has a mist discharging device and an ultrasonic vibration board integrated are respectively provided at upper portions of internal wall surfaces. Furthermore, outside the room 100 targeted to be decontaminated, there are provided an external storage tank 122, a weighing device 123 and solution supply pipes 124a to 124g and solution supply pumps 125a to 125g for supplying the hydrogen peroxide solution to the fixed decontamination devices 120a to 120g.

In FIG. 8, the hydrogen peroxide solution mist is discharged from the fixed decontamination devices 120a to 120g and is diffused inside the room 100 targeted to be decontaminated by ultrasonic waves. It is to be noted that the fixed decontamination devices 120a to 120g illustrated in FIG. 8 are all fixed types, and are not mobile decontamination apparatuses as in the second embodiment.

Although FIG. 8 is a top view diagram and does not illustrate details, the hydrogen peroxide mist discharged from the fixed decontamination devices 120a to 120g falls while diffusing in a parabola. In addition, the hydrogen peroxide mist has particle size distribution, and the hydrogen peroxide mist having large particle sizes (not illustrated) cannot sufficiently reach the position far away from each fixed decontamination devices 120a to 120g, wetting a floor surface in the vicinity thereof (not illustrated) . On the other hand, the hydrogen peroxide mist having small particle sizes similarly does not sufficiently reach the center portion of each chamber 111a to 111d. Such a phenomenon is likely to occur in a room targeted to be decontaminated having a complicated chamber shape.

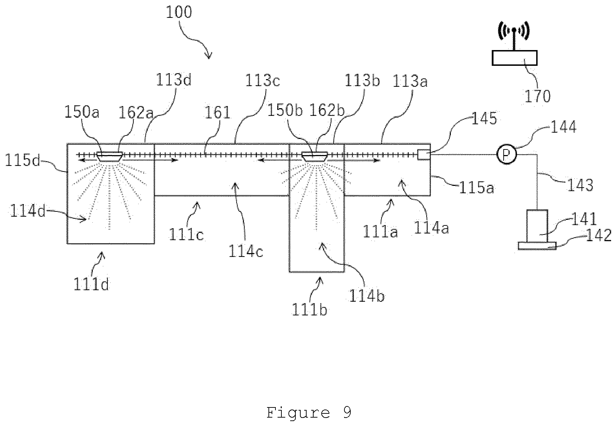

In contrast, FIG. 9 is a top view diagram of an internal cross section illustrating a state where the room targeted to be decontaminated in the second embodiment is decontaminated by the mobile decontamination apparatus according to the present invention. In FIG. 9, a content of the room 100 targeted to be decontaminated and the like is the same as those in FIG. 8, and the same reference signs are used therefor. It is to be noted that, in FIG. 9, the fixed decontamination device provided in the conventional decontamination method illustrated in FIG. 8 is not provided. Instead, a mobile decontamination apparatus 130 is provided at an upper portion of internal wall surface of each inside of the back surface walls 113a to 113d (inside work areas 114a to 114d) respectively formed in the chambers 111a to 111d and at the outside of the room 100 targeted to be decontaminated.

The mobile decontamination apparatus 130 includes a decontamination solution supply device 140, a decontamination solution discharging device 150, an indoor mobile device 160, and an operation control device 170. In FIG. 9, two decontamination solution discharging devices 150a, 150b and two traveling devices 162a, 162b are provided in the mobile decontamination apparatus 130, but the number of each device may be one.

The decontamination solution supply device 140 includes an external storage tank 141, a weighing device 142 and a solution supply pipe 143, a solution supply pump 144, and a solution supply station 145. The external storage tank 141, the weighing device 142, and the solution supply pipe 143 and the solution supply pump 144 are provided outside the room 100 targeted to be decontaminated. In contrast, the solution supply station 145 is provided at the upper portion of the internal wall surface inside the side face wall 115a (inside the work area 114a) of the chamber 111a. The external storage tank 141 and the solution supply station 145 are communicated with each other through the solution supply pipe 143. Consequently, a predetermined amount of the hydrogen peroxide solution weighed by the weighing device 142 is supplied to the solution supply station 145 by the solution supply pump 144.

The decontamination solution discharging devices 150a, 150b discharges the hydrogen peroxide mist while moving inside the respective work areas 114a to 114d of the respective chamber 111a to 111d. The structure of the decontamination solution discharging devices 150a, 150b is the same as that of the above-described first embodiment.

The indoor mobile device 160 includes a travel path 161 and traveling devices 162a, 162b. The travel path 161 is horizontally provided from the side face wall 115a of the chamber 111a to the side face wall 115d of the chamber 111d, at the upper portion of the internal wall surface inside the respective back surface walls 113a to 113d (inside the respective work areas 114a to 114d) of the respective chambers 111a to 111d. One end (side face wall 115a side) of the travel path 161 is connected to the solution supply station 145. The traveling devices 162a, 162b are integrally bonded respectively to back surfaces of the decontamination solution discharging devices 150a, 150b, and causes the decontamination solution discharging devices 150a, 150b to travel between the side face wall 115a to the side face walls 115d along the travel path 161. The structure and traveling mechanism of the travel path 161 and the traveling devices 162a, 162b are the same as those of the above-described first embodiment.

The operation control device 170 controls each operation of the weighing device 142 and the solution supply pump 144 in the decontamination solution supply device 140, a driving circuit (the same as that of the first embodiment) of the decontamination solution discharging devices 150a, 150b, the traveling devices 162a, 162b in the indoor mobile device 160, and the like, and cooperation operations thereof. The control performed by the operation control device 170 is the same as that of the above-described first embodiment.

In accordance with such a configuration, in the mobile decontamination apparatus according to the second embodiment, the room targeted to be decontaminated having a complicated shape of the chamber, which is a target for decontamination, where the plurality of isolator devices are connected can be efficiently decontaminated by the single mobile decontamination apparatus. Moreover, since the amount of hydrogen peroxide solution to be supplied to the decontamination solution discharging device can be accurately controlled to the necessary minimum, efficient decontamination can be performed corresponding to the size of the the room targeted to be decontaminated. Furthermore, since a precise amount of hydrogen peroxide mist can be supplied in this way, a sufficient decontamination effect can be obtained even with the minimum amount of decontamination solution required, achieving efficient use of the decontamination solution.

Accordingly, in the second embodiment, it is possible to provide a mobile decontamination apparatus capable of uniformly and completely decontaminating a room targeted to be decontaminated, equipment in a room, and the like, and particularly effective when decontaminating an inside of the room targeted to be decontaminated having a large area or decontaminating insides of a plurality of rooms targeted to be decontaminated.

### <Third Embodiment>

The following describes a case in which a plurality of mobile decontamination units are used to efficiently decontaminate a room targeted to be decontaminated composed of a very large-sized sterile isolator, for example, to which equipment for filling pharmaceutical products into vials or the like, equipment for freeze-drying the pharmaceutical products filled in the vials, and the like are connected, in pharmaceutical manufacturing fields.

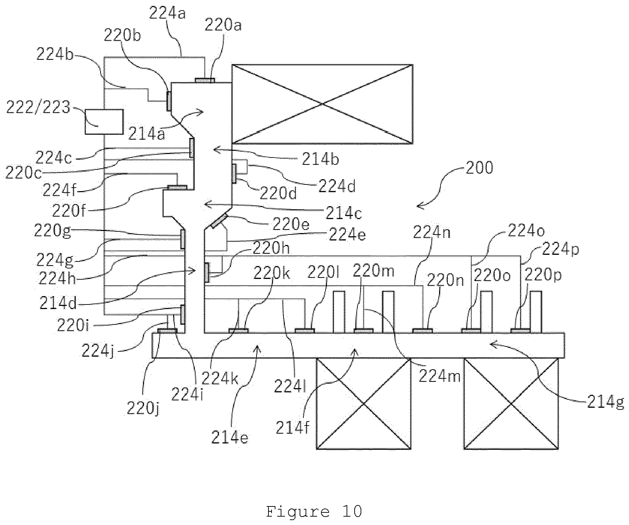

First, a case will be described in which a room targeted to be decontaminated which is composed of the above-described very large-sized sterile isolator is decontaminated by the conventional method. FIG. 10 is a top view diagram of an internal cross section illustrating a state in which a room targeted to be decontaminated in a third embodiment is decontaminated by a conventional method. In FIG. 10, the room 200 targeted to be decontaminated is bent in an L-shape as a whole, and is in a state where the shape of each work area 214a to 214g is also complicated. The descriptions of devices and articles installed in each work area, supply and exhaust blowers in each work area, and freeze-drying equipment connected to each work area 214a to 214g are omitted.

16 fixed decontamination devices 220a to 220p, each of which has a mist discharging device and an ultrasonic vibration board integrated are provided at an upper portion of an internal wall surface in the room 200 targeted to be decontaminated. Furthermore, outside the room 200 targeted to be decontaminated, there are provided an external storage tank 222, a weighing device 223 and solution supply pipes 224a to 224p and solution supply pumps (not illustrated) for supplying the hydrogen peroxide solution to the fixed decontamination devices 220a to 220p.

In FIG. 10, the hydrogen peroxide solution mist is discharged from the fixed decontamination devices 220a to 220p and is diffused inside the room 200 targeted to be decontaminated by ultrasonic waves. It is to be noted that the fixed decontamination devices 220a to 220p illustrated in FIG. 10 are all fixed types, and are not mobile decontamination apparatuses as in the third embodiment.

Although FIG. 10 does not illustrate details, the hydrogen peroxide mist discharged from the fixed decontamination devices 220a to 220p falls while diffusing in a parabola. Even in the case in which the 16 fixed decontamination devices 220a to 220p are provided, there is a possibility that the hydrogen peroxide mist having large particle sizes (not illustrated) cannot sufficiently reach to the position away from each fixed decontamination device 220a to 220p, wetting a floor surface in the vicinity thereof (not illustrated). On the other hand, there is also a possibility that the hydrogen peroxide mist having small particle sizes (not illustrated) cannot sufficiently reach the center portion of each work area 214a to 214g. Such a phenomenon is likely to occur in a room targeted to be decontaminated composed of a very large-sized sterile isolator.

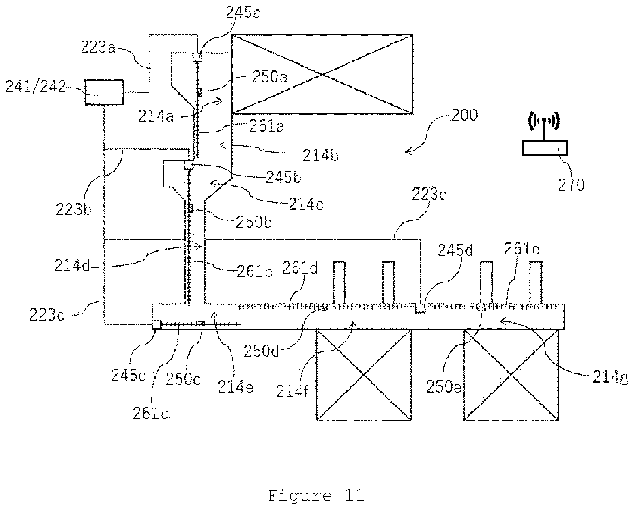

In contrast, FIG. 11 is a top view diagram of an internal cross section illustrating a state where the room targeted to be decontaminated in the third embodiment is decontaminated by the mobile decontamination apparatus according to the present invention. In FIG. 11, a content of the room 200 targeted to be decontaminated and the like is the same as those in FIG. 10, and the same reference signs are used therefor. It is to be noted that, in FIG. 11, the fixed decontamination device provided in the conventional decontamination method illustrated in FIG. 10 is not provided. Instead, five mobile decontamination apparatuses 230a to 230e are provided at an upper portion of the internal wall surface all over the room 200 targeted to be decontaminated (work areas 214a to 214g), and outside the room 200 targeted to be decontaminated. The mobile decontamination apparatuses 230a to 230e have a decontamination solution supply device 240 (shared by five devices), decontamination solution discharging devices 250a to 250e, indoor mobile devices 260a to 260e, and an operation control device 270 (shared by five devices).

The decontamination solution supply device 240 includes an external storage tank 241, a weighing device 242 and solution supply pipes 243a to 243d, a solution supply pump (not illustrated), and solution supply stations 245a to 245d. The external storage tank 241, the weighing device 242, and the solution supply pipes 243a to 243d and the solution supply pump (not illustrated) are provided outside the room 200 targeted to be decontaminated. On the other hand, the solution supply stations 245a to 245d are provided to be distributed at upper portions of internal wall surfaces in the work areas 214a to 214g (refer to FIG. 11). The external storage tank 241 and the solution supply stations 245a to 245d are communicated with each other through the solution supply pipes 243a to 243d. Consequently, a predetermined amount of the hydrogen peroxide solution weighed by the weighing device 242 is supplied to the solution supply stations 245a to 245d by the solution supply pump (not illustrated).

The five decontamination solution discharging devices 250a to 250e discharge hydrogen peroxide mist while moving inside the room 200 targeted to be decontaminated. The structure of the decontamination solution discharging devices 250a to 250e is the same as that of the above-described first embodiment.

The five indoor mobile devices 260a to 260e respectively includes five travel paths 261a to 261e and traveling devices 262a to 262e. The travel paths 261a to 261e are horizontally provided all over the upper portion of the internal wall surface in the work areas 214a to 214g (refer to FIG. 11) . The travel paths 261a to 261e are respectively connected to the solution supply stations 245a to 245d at predetermined portions (refer to FIG. 11) . The traveling devices 262a to 262e are integrally bonded respectively to back surfaces of the decontamination solution discharging devices 250a to 250e, and causes the decontamination solution discharging devices 250a to 250e to respectively trave over the upper portion of the internal wall surface in the work areas 214a to 214g along the travel paths 261a to 261e. The structure and traveling mechanism of the travel paths 261a to 261e and the traveling devices 262a to 262e are the same as those of the above-described first embodiment.

The operation control device 270 (shared by five devices) controls each operation of the weighing device 242 and the solution supply pump (not illustrated) in the decontamination solution supply device 240, a driving circuit (the same as that of the first embodiment) of the decontamination solution discharging devices 250a to 250e, the traveling devices 261a to 261e in the indoor mobile devices 260a to 260e, and the like, and cooperation operations thereof. The control performed by the operation control device 270 is the same as that of the above-described first embodiment.

In accordance with such a configuration, in the mobile decontamination apparatus according to the third embodiment, the plurality of mobile decontamination units can efficiently decontaminate the room to be decontaminated composed of a very large-sized sterile isolator, for example, to which equipment for filling pharmaceutical products into vials or the like, equipment for freeze-drying the pharmaceutical products filled in the vials, and the like are connected, in pharmaceutical manufacturing fields. Moreover, since the amount of hydrogen peroxide solution to be supplied to the decontamination solution discharging device can be accurately controlled to the necessary minimum, efficient decontamination can be performed corresponding to the size of the the room targeted to be decontaminated. Furthermore, since a precise amount of hydrogen peroxide mist can be supplied in this way, a sufficient decontamination effect can be obtained even with the minimum amount of decontamination solution required, achieving efficient use of the decontamination solution.

Accordingly, in the third embodiment, it is possible to provide a mobile decontamination apparatus capable of uniformly and completely decontaminating a room targeted to be decontaminated, equipment in a room, and the like, and particularly effective when decontaminating an inside of the room targeted to be decontaminated having a large area or decontaminating insides of a plurality of rooms targeted to be decontaminated.

In addition, when the present invention is carried out, the following modification examples are shown without being limited to each of the embodiments.
(1) In each of the above-described embodiments, decontamination of rooms targeted to be decontaminated having a large area or a complicated shape has been described. However, it is not limited thereto, and may be used for decontamination of compact isolators, pass boxes, and the like.
(2) In each of the above-described embodiments, the hydrogen peroxide solution is supplied to the decontamination solution discharging device in the mobile decontamination apparatus through the solution supply station. However, it is not limited thereto, and it may be configured to directly supply the decontamination solution discharging device from the external storage tank.
(3) In each of the above-described embodiments, the rechargeable type batteries are used as the power supply device (battery) for driving circuits. However, it is not limited thereto, and exchangeable cartridge-type batteries may be used for both.
(4) In each of the above-described embodiments, the cases have been described in which the mobile decontamination apparatus efficiently decontaminates the rooms targeted to be decontaminated which are the chamber that is wide and has a large planar aspect ratio, the room targeted to be decontaminated having the complicated chamber shape to which the plurality of isolator devices are connected, and the very large-sized sterile isolator. However, it is not limited thereto, and the mobile decontamination apparatus may be used to efficiently decontaminate the inside of a relatively narrow space, such as a pass box.
(5) In each of the above-described embodiments, it is used as a mobile decontamination apparatus, but it can be diverted to a leak test of HEPA filters by replacing the decontamination solution discharging device in the present device with a suction probe of a particle counter.
(6) In each of the above-described embodiments, the travel path of the indoor mobile device is provided at the upper portion of the internal wall surface in the chamber. However, it is not limited thereto, and the travel path may be provided at a center portion of a ceiling portion in the chamber. In this case, the mist discharge port of the decontamination solution discharging device may be provided not only at the front side of the device but also at the front, back, and bottom sides.
(7) In each of the above-described embodiments, the travel path of the indoor mobile device is provided in the upper portion of the internal wall surface in the chamber. However, it is not limited thereto, and the travel path may be provided at any position, such as such as below a wall of the chamber or on a surface of a workbench.
(8) In each of the above-described embodiments, the driving circuit provided in the decontamination solution discharging device controls the operation of the mist generating device. On the other hand, the drive device for controlling the drive of the traveling device shall be provided in the traveling device. However, it is not limited thereto, and it may be configured that the driving circuit for controlling the drive of the traveling device is provided inside the decontamination solution discharging device. Furthermore, the driving circuit for controlling both of the mist generating device and the drive device may be provided inside the decontamination solution discharging device or inside the drive device.
(9) In each of the above-described embodiment, the power supply device (battery) provided in the decontamination solution discharging device shall be configured to operate the driving circuit. However, it is not limited thereto, and the power supply device for operating the driving circuit may be provided inside the traveling device. Furthermore, the power supply device for operating both of the mist generating device and the drive device may be provided inside the decontamination solution discharging device or inside the drive device.

### REFERENCE SIGNS LIST

10...Isolator device,
11...Chamber,
11a to 11d, 112a to 112d, 113a to 113d...Wall surface,
12...Air supply blower,
13...Air exhaust blower,
14, 114a to 114d, 214a to 214g...Work area,
15...Air supply fan,
16...Filter unit,
17...Screen mesh straightening plate,
18a to 18c...Article,
20a to 20c...Mist discharging device,
21a to 21c...Ultrasonic vibration board,
22, 41, 122, 141, 222, 241...External storage tank,
23, 123, 223...Weighing device,
24a to 24c, 43, 124a to 124g, 143, 224a to 224p, 243a to 243d...Solution supply pipe,
25a to 25c, 44, 144, 125a to 125g...Solution supply pump,
30, 130, 230a to 230e...Mobile decontamination apparatus,
40, 140, 240...Decontamination solution supply device,
42, 142, 242...Weighing device,
45, 145, 245a to 245d...Solution supply station,
50, 150a, 150b, 250a to 250e...Decontamination solution discharging device,
51...Mist generating device,
51a...Piezoelectric vibrator,
51b...Porous vibration plate,
52...Driving circuit,
53...Battery (power supply device),
54...Hydrogen peroxide solution tank (decontamination solution tank) ,
54a...Narrow tube,
55...Mist discharge port,
60, 160, 260a to 260e...Indoor mobile device,
61, 161, 261a to 261e...Travel path,
62, 162a, 162b, 262a to 262e...Traveling device,
70, 170, 270...Operation control device,
100, 200...Room targeted to be decontaminated,
111a to 111d...Chamber, and
120a to 120g, 220a to 220p...Fixed decontamination device.

## Claims

1. A mobile decontamination apparatus configured to decontaminate an inside of a room targeted to be decontaminated using a decontamination agent mist,
the mobile decontamination apparatus comprising a decontamination solution discharging device and an indoor mobile device, **characterized in that**
the decontamination solution discharging device comprises:
a decontamination solution tank;
one or more mist generating devices configured to generate a decontamination agent mist from a decontamination solution;
a driving circuit configured to control a drive of the mist generating device; and
one or more mist discharge ports configured to discharge the decontamination agent mist generated by the mist generating device into the inside of the room targeted to be decontaminated, and
the indoor mobile device comprises:
a travel path disposed inside the room targeted to be decontaminated; and
a traveling device configured to cause the decontamination solution discharging device to travel along the travel path.

2. The mobile decontamination apparatus according to claim 1, further comprising
a decontamination solution supply device including an external storage tank configured to store the decontamination solution in advance, and a solution supply pipe for supplying the decontamination solution between the external storage tank and the decontamination solution discharging device, **characterized in that**
the decontamination solution supply device comprises a weighing device and a solution supply pump, and is configured to weigh and supply a predetermined amount of the decontamination solution to the decontamination solution discharging device through the solution supply pipe.

3. The mobile decontamination apparatus according to claim 2, further comprising
an operation control device configured to control an operation of the decontamination solution supply device, the decontamination solution discharging device, and the indoor mobile device, **characterized in that**
the operation control device controls, in accordance with a program predetermined, the weighing device and the solution supply pump included in the decontamination solution supply device, to weigh a predetermined amount of decontamination solution and to adjust an amount of the solution supplied to the decontamination solution discharging device,
controls the driving circuit included in the decontamination solution discharging device to adjust an amount of the mist discharged, and
controls the traveling device included in the indoor mobile device to adjust a position of the decontamination solution discharging device inside the room targeted to be decontaminated,
thereby controlling the position of the room targeted to be decontaminated and an amount of the mist discharged at the position.

4. The mobile decontamination apparatus according to claim 3, further comprising
a solution supply station configured to temporarily store the predetermined amount of decontamination solution disposed on a pipeline of the solution supply pipe for communicating between the external storage tank and the decontamination solution discharging device, **characterized in that**
the operation control device controls a solution supply from the decontamination solution supply device to the solution supply station and a solution supply from the solution supply station to the decontamination solution discharging device.

5. The mobile decontamination apparatus according to claim 1, **characterized in that**
the decontamination solution tank included in the decontamination solution discharging device is an exchangeable cartridge-type tank filled with a predetermined amount of decontamination solution.

6. The mobile decontamination apparatus according to claim 1, **characterized in that**
the decontamination solution discharging device or the indoor mobile device includes an exchangeable cartridge-type power supply device.

7. The mobile decontamination apparatus according to any one of claims 1-6, **characterized in that**
the mist generating device is an ultrasonic atomizer comprising a piezoelectric vibrator and a porous vibration plate with a plurality of micropores for atomizing the decontamination solution by vibration of the piezoelectric vibrator, the micropores provided so as to pass through the porous vibration plate between front and back surfaces thereof, wherein
the porous vibration plate constitutes the mist discharge port.
